# EUROPEAN PATENT APPLICATION

(11) **EP 3 909 578 A1**
(43) Date of publication of application: **17.11.2021**
(21) Application number: 20382386.9
(22) Date of filing: 12.05.2020
(51) Int. Cl.: A61K 31/4166, A61P 31/14, A61K 31/4178

(54) **ANTIVIRAL COMPOSITION COMPRISING EEYARESTATIN I**

(71) Applicant: Universidad de Castilla la Mancha, 13071 Ciudad Real (ES)
(72) Inventor: ARIAS ESTEBAN, Armando, 02008 Albacete (ES); MAS LÓPEZ, Antonio, 02008 Albacete (ES)
(74) Representative: Hoffmann Eitle

(57) **Abstract**

Antiviral composition. The present invention refers to eeyarestatin I, or antiviral compositions comprising thereof, for use in the treatment of viral infections caused by a virus member of the *Flaviviridae* family.

## Description

### FIELD OF THE INVENTION

The present invention refers to the medical field. Particularly, it refers to eeyarestatin I, or antiviral compositions comprising thereof, for use in the treatment of viral infections caused by a virus member of the *Flaviviridae* family.

### STATE OF THE ART

*Flavivirus* is a genus of viruses in the family *Flaviviridae,* which comprises pathogens that are primarily spread through arthropod vectors (mainly ticks and mosquitoes). Humans and other animals serve as natural hosts.

There is a growing number of human and animal diseases caused by flaviviruses transmitted by insects, all of them belonging to the genus *Flavivirus.* The most relevant viruses from a human point of view are dengue, Zika, West Nile, yellow fever, Japanese encephalitis and tick-borne encephalitis. Zika virus is a new emerging threat to global health with estimates suggesting >10 million people were infected during recent epidemics in the Americas and Southeast Asia. Zika virus infection has been linked to thousands of cases of brain development defects in neonates (microcephaly) and Guillain-Barre syndrome in adults. Dengue virus infects several hundred million people every year, causing large numbers of fatalities (25,000) and cases of severe haemorrhagic disease associated (500,000). In temperate regions such as North America and Southern and Central Europe there is an increase in the incidence of human disease caused by zoonotic flaviviruses. Specifically, West Nile and Usutu viruses, which both have birds as their natural hosts, are responsible for a growing number of cases of neurologic disease in humans.

In addition to arthropod-transmitted viruses, other members of the *Flaviviridae* family not transmitted by vectors are also relevant to public and animal health (e.g. pestiviruses, pegiviruses). Notably, hepatitis C virus (genus *Hepacivirus*) chronically infect ∼ 70 million people worldwide and causes an estimate number of 400,000 deaths annually.

As explained above, there are yet no licenced antiviral drugs to treat disease caused by viruses belonging to the genus *Flavivirus,* particularly mosquito-borne viruses. The only treatments available are antipyretics and analgesics that help alleviate the clinical symptoms associated with infection but not to control and/or cure the infection.

So, there is an unmet medical need of finding antiviral compositions which can be effectively used for the treatment of infections caused by viruses of the *Flaviviridae* family, particularly mosquito-borne viruses.

The present invention is focused on solving this problem, and consequently it is herein provided an antiviral composition for the treatment of infections caused by viral members of the *Flaviviridae* family, particularly mosquito-borne viruses.

### DESCRIPTION OF THE INVENTION

### Brief description of the invention

The present invention refers to eeyarestatin I (CAS number 412960-54-4), or antiviral compositions comprising thereof, for use in the treatment of viral infections caused by a virus member of the *Flaviviridae* family.

Such as it is explained above, there are no antiviral compounds licenced for the treatment of flaviviruses transmitted by mosquitoes, particularly for the treatment of infections caused by Zika virus or Usutu virus.

The inventors of the present invention have found an antiviral activity associated with eeyarestatin I upon two flaviviruses, Zika virus and Usutu virus. A dose- and time-dependent loss of viral infectivity in particles incubated in the presence of eeyarestatin I is herein shown, which supports the virucidal activity of the compound.

Although the present invention offers results mainly with respect to Zika virus and Usutu virus, kindly note that these viruses have been precisely chosen as proof-of-concept for the entire *Flaviviridae* family. Since Zika virus and Usutu virus are relatively genetically distant, we argue that the results obtained in the present invention can be extrapolated to other members of the *Flaviviridae* family, particularly mosquito-borne viruses. Zika and Usutu viruses belong to two different serocomplex groups within the genus *Flavivirus:* the Japanese encephalitis virus group (Usutu virus) and the Spondweni virus group (Zika virus). Thus, the fact that eeyarestatin I is similarly effective against viruses from different subgroups suggests a broad antiviral activity against other members of the same genus (*Flavivirus*), and the same family (*Flaviviridae*).

Therefore, eeyarestatin I is herein proposed as an antiviral drug against flaviviruses, particularly those flaviviruses transmitted by mosquitoes (e.g. dengue, Zika, Usutu, West Nile, yellow fever viruses). Moreover, it is also herein suggested the use of eeyarestatin I as a direct-acting antiviral (DAA) against hepatitis C virus particle (envelope and/or structural proteins), also a member of the *Flaviviridae* family, to complement and improve current multidrug cocktail therapies based on non-structural protein inhibitors only (Bhatia and Gupta, 2020).

So, the first embodiment of the present invention refers to eeyarestatin I for use in the treatment of viral infections caused by a virus member of the *Flaviviridae* family.

In a preferred embodiment, the present invention refers to eeyarestatin I for use in the treatment of viral infections caused by a virus member of the *Flavivirus* genus.

In a preferred embodiment, the present invention refers to eeyarestatin I for use in the treatment of viral infections caused by mosquito-borne viruses.

In a preferred embodiment, the present invention refers to eeyarestatin I for use in the treatment of viral infections caused by Zika virus or Usutu virus.

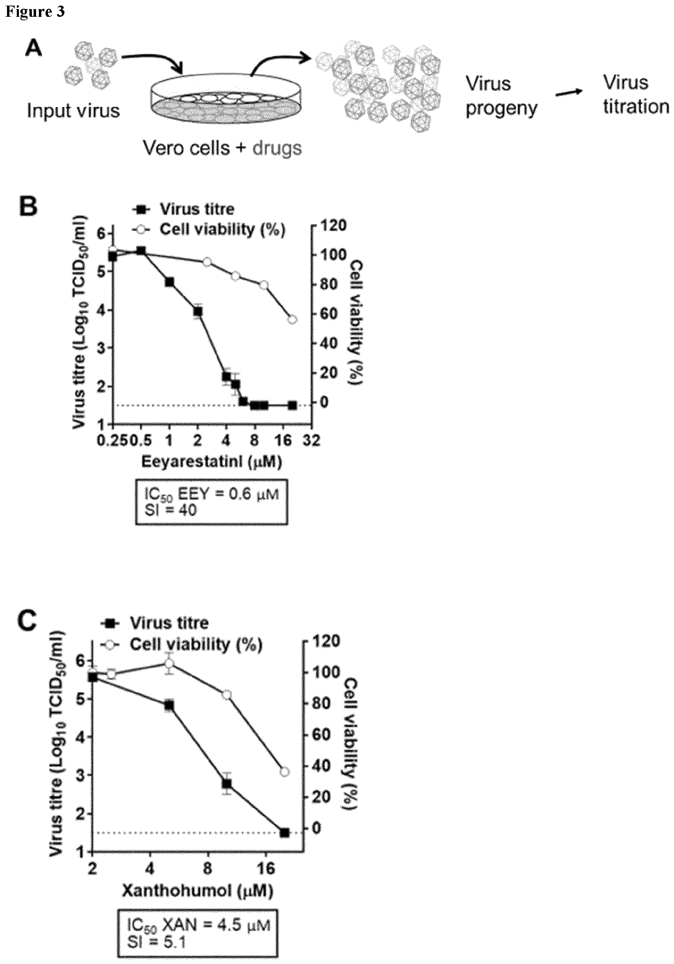

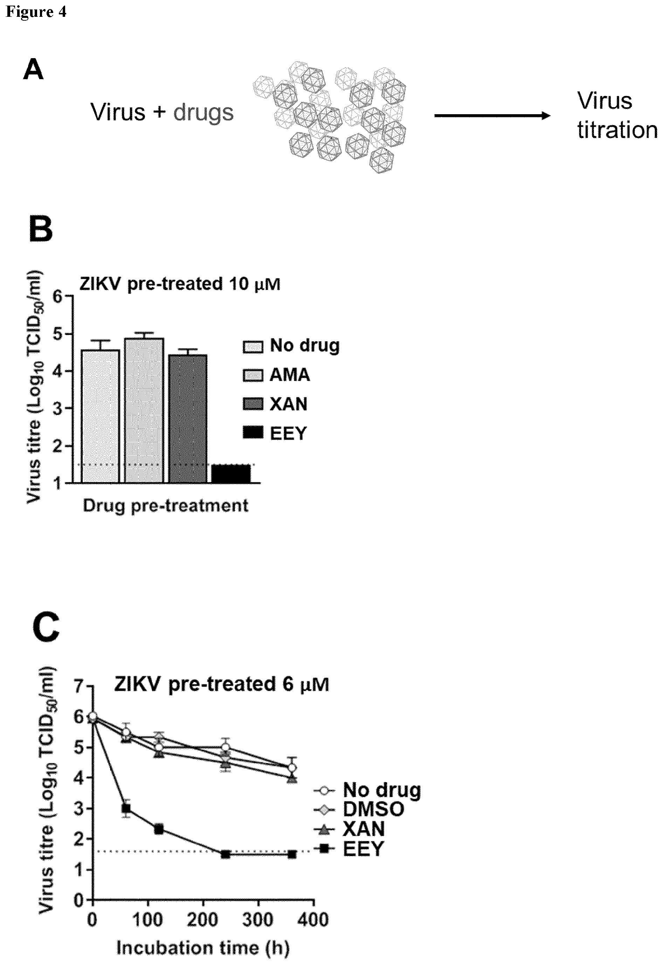

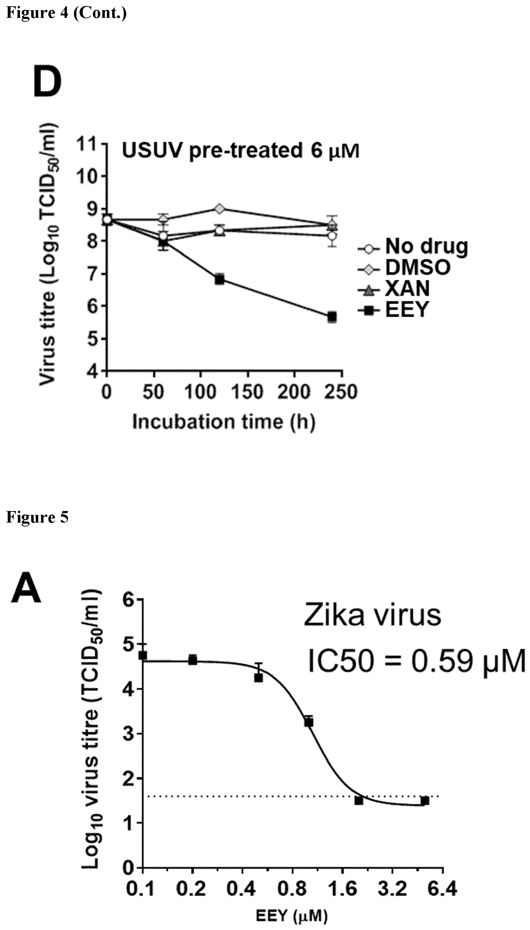

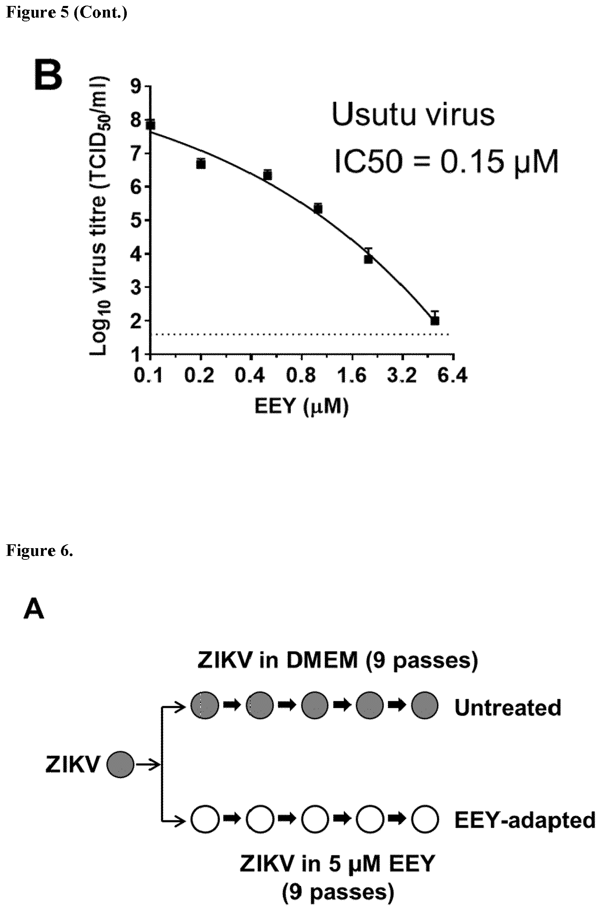

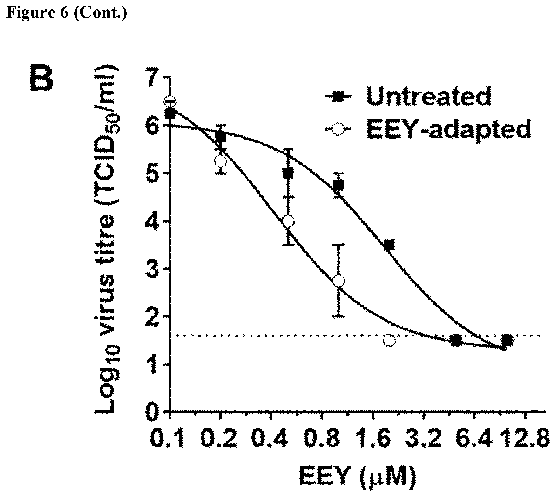

In a preferred embodiment, the antiviral activity is exerted by directly interacting with the viral particle during the extracellular viral phase. Thus, the inventors of the present invention propose a new mechanism of action by which eeyarestatin I exerts its virucidal activity by directly interacting with the viral particle during the extracellular viral phase, causing neutralization, destabilization, inactivation and/or disruption of the viral particle per se, leading to reduction in infectivity. Kindly note that the fact that a compound presents antiviral activity inside the host cells (for instance by blocking virus replication) does not inherently mean that this compound will effectively act against the viral particle itself. This is supported by the experimental data presented in **Figure 4** below, wherein it is shown that the compound xanthohumol (an inhibitor related to eeyarestatin I as both target the same host intracellular protein) exhibits antiviral activity in infected cells **(****Figure 3****)** but does not manifest virucidal activity over viral particles **(****Figure 4****),** let alone over a virus member of the *Flaviviridae* family, virus member of the *Flavivirus* genus, mosquito-borne viruses or, specifically, Zika virus or Usutu virus. In fact, as cited above, xanthohumol exhibits antiviral activity in infected cells but does not have virucidal activity on viral particles of a virus member of the *Flaviviridae* family, virus member of the *Flavivirus* genus, mosquito-borne viruses or, specifically, Zika virus or Usutu virus. An additional advantage with respect to other classic antivirals is that eeyarestatin I seems not to drive the selection for drug resistance *[*Bhatia, M., Gupta, E., 2020. Emerging resistance to directly-acting antiviral therapy in treatment of chronic Hepatitis C infection-A brief review of literature. J. Fam. Med. Prim. Care 9, 531. https://doi.org/10.4103/JFMPC.JFMPC_943_19]. This is supported by data presented in **Figure 6****.** Here, we showed that a Zika virus population obtained after extensive replication in the presence of eeyarestatin I is equally sensitivity to the virucidal activity associated with this drug than the parental strain. To this aim, Zika virus was propagated in cell culture during nine consecutive passages in the presence of eeyarestatin I. The resulting population rescued from the ninth passage showed no increased resistance to eeyarestatin I virucidal activity **(****Figure 6****).**

The second embodiment of the present invention refers to a pharmaceutical composition, specifically an antiviral composition, comprising eeyarestatin I and, optionally, pharmaceutically acceptable carriers or excipients, for use in the treatment of viral infections caused by a virus member of the *Flaviviridae* family.

In a preferred embodiment, the present invention refers to an antiviral composition comprising eeyarestatin I and, optionally, pharmaceutically acceptable carriers or excipients, for use in the treatment of viral infections caused by a virus member of the *Flavivirus* genus.

In a preferred embodiment, the present invention refers to an antiviral composition comprising eeyarestatin I and, optionally, pharmaceutically acceptable carriers or excipients, for use in the treatment of viral infections caused by mosquito-borne viruses.

In a preferred embodiment, the present invention refers to an antiviral composition comprising eeyarestatin I and, optionally, pharmaceutically acceptable carriers or excipients, for use in the treatment of viral infections caused by Zika virus or Usutu virus.

The third embodiment of the present invention refers to a method for treating a person infected by a virus member of the *Flaviviridae* family, by a virus member of the *Flavivirus* genus, by mosquito-borne viruses or, specifically, by Zika virus or Usutu virus which comprised the administration to the subject of a therapeutically effective dose or amount of eeyarestatin I or a composition comprising thereof.

For the purpose of the present invention the following terms are defined:
- By the term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of a composition comprising eeyarestatin I is intended an amount that, when administered as described herein, brings about a positive therapeutic response in a subject having an infection caused by a virus member of the *Flaviviridae* family, by virus member of the *Flavivirus* genus, by mosquito-borne viruses or, specifically, by Zika virus or Usutu virus. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

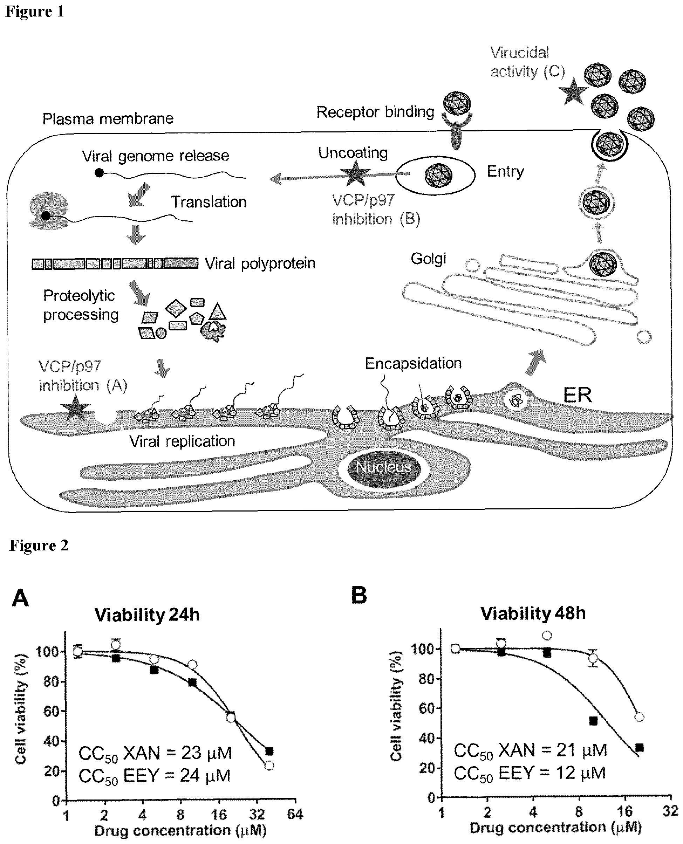

**Figure 1****. Schematic representation of flavivirus life cycle and steps inhibited by eeyarestatin I.** The antiviral effect observed during treatment with eeyarestatin I can be due to the inhibition of at least three different steps of the life cycle (indicated with a red star and highlighted in red letters). **A,** Inhibition of the valosin-containing protein / p97 ATPase (VCP/p97) and/or ERAD pathway indirectly affects virus replication on ER factories. **B,** Inhibition of VCP/p97 affects virus uncoating. **C,** Novel mechanism of action described in this invention: eeyarestatin I reduces virus particle infectivity before entering the cell (discovery patented here).
**Figure 2****. Cell viability after exposure to VCP/p97 inhibitors.** Viability of Vero (African green monkey kidney fibroblasts) cells treated with VCP/p97 inhibitors xanthohumol (XAN) and eeyarestatin I (EEY) during 24 **(A)** or 48 hours **(B).** Cellular viability is represented as the percentage of metabolically active cells relative to mock-treated cells (CellTiter-Blue Cell Viability Assay, Promega). There are given the 50% cytotoxicity concentration (CC₅₀) values for each drug and exposure time tested.
**Figure 3****. VCP/p97 drugs Xanthohumol and eeyarestatin I inhibit virus replication in cell culture (Vero cells).** Virus titres measured by 50% tissue culture infectious dose (TCID₅₀) in the supernatants of Zika virus-infected cells. **A,** Schematic representation of the assay. A monolayer of Vero cells was inoculated with Zika virus at a multiplicity of infection (MOI) of 1 TCID₅₀ per cell (input). After 1 hour of incubation at 37°C, to allow virus adsorption, the supernatant was removed, the cells washed, and fresh media containing each drug at the concentrations indicated was added. At 24 hours post infection the cellular supernatants were collected (virus progeny) and the virus yields quantified by TCID₅₀ (virus titration). **B-C**, Changes in virus titres and cell viability in cultures treated with increasing concentrations of eeyarestatin I **(B)** and xanthohumol **(C)** for 24 hours. The 50% inhibitory concentration (IC₅₀) and the selectivity index (SI) values are provided for each drug. Selectivity index is calculated as the ratio of CC₅₀ (provided in **Figure 2**) and IC₅₀.
**Figure 4****. Eeyarestatin I but not xanthohumol exhibit virucidal activity against Zika virus and Usutu virus in a cell-independent context. A,** Schematic representation of the assay. The virus sample is directly incubated at 37°C in the presence of increasing concentrations of drugs. The treated sample is collected at different time points and directly titrated by TCID₅₀. **B-D** Decrease in infectivity observed in a virus sample treated with amantadine (AMA), xanthohumol (XAN) or eeyarestatin I (EEY) and compared to a virus sample untreated (no drug) or mock-treated with vehicle (DMSO). **B,** Zika virus was incubated during 4 hours at 37°C in the presence of EEY (10 µM), XAN (10 µM), AMA (800 µM) or in the absence of drugs. **C,** Changes in the infectivity along time of a Zika virus sample incubated at 37°C in the presence of 6 µM EEY or XAN, or in the absence of drugs (No drug, DMSO). **D,** Changes in Usutu virus infectivity along time for a sample incubated at 37°C in the presence of 6 µM EEY or XAN, or in the absence of drugs (No drug, DMSO).
**Figure 5****. Eeyarestatin I exerts virucidal activity in a dose-dependent manner.** Eeyarestatin I exhibits virucidal activity on both Zika **(A)** and Usutu **(B)** viruses. Eeyarestatin I half maximum inhibitory concentration (IC₅₀) is <1 µM for both Zika **(A)** and Usutu **(B)** viruses.
**Figure 6****. Prolonged treatment with eeyarestatin I do not select for Zika virus variants with reduced susceptibility to its virucidal activity. A,** Schematic representation of the procedure to select adapted virus. In each virus passage, 100 µl of neat virus from the previous passage are inoculated to a monolayer of 10⁵ Vero cells. After cell wash, the monolayer is incubated at 37°C and 5% CO2 in 1 ml of media (DMEM, 10% foetal bovine serum, 100 units penicillin-streptomycin) in the presence (EEY-adapted) or absence (untreated) of 5 µM eeyarestatin I. At 48 hours post-infection, the cellular supernatants are collected, titrated, and employed for the following virus passage. This process is repeated for a total of nine viral transfers. **B,** the cellular supernatants of Zika virus passaged during nine consecutive transfers (p9) in the presence (EEY-adapted) or absence (untreated) of 5 µM eeyarestatin I were titrated. Then, an equivalent amount of EEY p9 and Untreated p9 viruses were mixed with increasing concentrations of eeyarestatin I, and incubated at 37°C for 4 hours, as previously described in **Figure 4****.** The IC₅₀ values of eeyarestatin I were 0.13 and 0.31 µM for EEY-adapted and untreated Zika viruses, respectively.

### Detailed description of the invention

A detailed description of the invention is herein provided by means of the following examples, without the intention of limiting its scope of protection.

### Example 1. Eeyarestatin I can act as an inhibitor of at least three steps of the virus life cycle.

Eeyarestatin I is a specific inhibitor of an AAA ATPase named valosin-containing protein (VCP), also known as p97. VCP/p97 plays a major role assisting to pathways of degradation of proteins associated with the endoplasmic reticulum (ERAD). In this process, VCP/p97 recruits and ushers ubiquitinated proteins, which have been previously retrotranslocated from the ER to the cytosol (through the translocon Sec61), to the proteasome where they are degraded.
There is mounting evidence supporting that ERAD, and thus VCP/p97 play a proviral role during infection **(****Figure 1****),** which has encouraged the use of drugs targeting these factors **(****Figure 1****).** Most RNA viruses, including the flaviviruses, replicate their genomes in factories tightly associated with ER membranes, supporting a connection between viral genome replication and cellular responses to stress such as ERAD
In addition to its proviral activity connected to ERAD, it has also been recently documented that VCP/p97 assists viral genome uncoating during virus particle entry in the cell. Inhibition of VCP/p97 resulted in decreased release of viral genomes into the cytoplasm, and therefore reduced levels of infected cells. In addition to these two possible antiviral activities of associated with the inhibition of VCP/p97 (ERAD proviral role and virus uncoating), here we provide evidence for a virucidal activity of eeyarestatin I on virus infectious particles which occurs extracellularly, and in the absence of VCP/p97 **(****Figure 1****).**

### Example 2. Different VCP/p97 inhibitors affect Zika virus replication in cell culture.

We have examined the antiviral activity associated with VCP/p97 inhibition by using two different drugs: xanthohumol and eeyarestatin I **(****Figure 2** and **Figure 3****).** Xanthohumol is a prenylated compound extracted from the hop plant which has been tested in several clinical trials; e.g. to examine its effect on the metabolic syndrome, to evaluate whether it prevents DNA damage *in vivo,* etc. Eeyarestatin I was identified as an inhibitor of ERAD-associated protein degradation during high-throughput screening of compounds, using a green fluorescent protein (GFP)-based reporter assay. Eeyarestatin I has not been yet clinically examined although its anticancer behaviour has been documented in mice. Here we show that both compounds inhibit Zika virus replication during infections in cell culture **(****Figure 3****).** These antiviral activities are attained at drug concentrations which have little or no effect on cellular viability, as evinced by selectivity indexes > 5 in both cases. To these assays, we infected monolayers of Vero cells at an MOI of 1, following protocols that we have previously documented *[*Bassi, M.R., Sempere, R.N., Meyn, P., Polacek, C., Arias, A., 2018. Extinction of Zika virus and Usutu virus by lethal mutagenesis reveals different patterns of sensitivity to three mutagenic drugs. Antimicrob. Agents Chemother. 62, e00380-18. https://doi.org/10.1128/MC00380-18]. After virus inoculation, we incubated the cell monolayers at 37°C for 1 hour to allow adsorption of viral particles to the cell. Then, unattached virus was removed, the cell monolayer washed, and fresh media containing different drug concentrations added. Thus, we conclude that any decrease in viral yields is possibly due to reduced intracellular viral genome replication as a result of the inhibition of VCP/p97 and its proviral roles associated.

### Example 3. Eeyarestatin I but not xanthohumol elicit virucidal activity upon Zika virus and Usutu virus prior to infection.

We have found an unexpected virucidal activity (loss of viral particle infectivity) for eeyarestatin I on Zika virus and Usutu virus **(****Figure 4****).** Pre-incubation of Zika or Usutu virus samples with eeyarestatin I (in the absence of cells) causes a significant drop in virus infectivity. To this assay, virus samples previously incubated with eeyarestatin I (in the absence of cells) were titrated in 50% tissue culture infectious dose assays (TCID₅₀). Hence, any antiviral activity observed here occurs in a VCP/p97-independent manner as no cells are involved during the virus-drug incubation period. Further supporting this hypothesis, we have found no virucidal activity for xanthohumol (another VCP/p97 inhibitor) or vehicle alone (DMSO). The loss in viral sample infectivity linked to pre-incubation with eeyarestatin I is time-dependent; under similar experimental conditions (eeyarestatin concentration, virus dose) we observed larger decreases in virus titre for samples exposed to the drug during longer periods **(****Figure 4****).** This was never observed for samples incubated with xanthohumol or DMSO. These observations further support that eeyarestatin I directly affects the virus particle infectivity and/or its integrity.

### Example 4. Eeyarestatin I is a potent virucidal compound.

The decay in infectivity for both Zika virus and Usutu virus is drug concentration-dependent which has allowed us to calculate the half maximal inhibitory concentration (IC₅₀) of eeyarestatin I **(****Figure 5****).** We have found that eeyarestatin I is a potent virucidal molecule with IC₅₀ values below 1 µM for both Zika (0.59 µM) and Usutu (0.15 µM) viruses. The resulting selectivity index, which measures how many times the antiviral efficacy exceeds the cellular toxicity is >40 for both viruses. To calculate the selectivity index, we divide the cytotoxic concentration 50% (CC₅₀) value **(****Figure 2****),** by the IC₅₀ value **(****Figure 5****).** These observations suggest that eeyarestatin I is highly effective and selective against these viruses, and further encourage its use as a therapeutic drug in the treatment of infection.

### Example 5. Sustained propagation of Zika virus in the presence of eeyarestatin I does not select for resistant variants with reduced virucidal sensitivity.

To further investigate the therapeutic potential of eeyarestatin I, we assessed whether prolonged treatment could lead to the selection of viruses with increased resistance to eeyarestatin I **(****Figure 6****).** To this aim, we passaged Zika virus during nine successive infections in Vero cells in the presence of 5 µM eeyarestatin I (each infection was allowed to proceed for 48 hours). This concentration is eight times larger than the inhibitory constant (IC₅₀) found for Zika virus **(****Figure 4****),** while it causes very modest toxicity in cell culture **(****Figure 2****).** Consequently, the viral population isolated after 9 passages have replicated in the presence of 5 µM eeyarestatin I for a total of 18 days (432 hours). A control Zika virus population was obtained after nine consecutive passages in the absence of drugs (untreated virus). We found that both Zika virus lineages (adapted and untreated) show similar sensitivity to eeyarestatin I. Indeed, the adapted population (passaged 9 times in the presence of eeyarestatin I) exhibited slightly larger sensitivity to this drug (IC50 of 0.13 uM) with respect to the untreated population (IC50 of 0.31 uM). These results suggest that eeyarestatin I is a highly efficacious drug that do not select for adaptive resistance mutations after prolonged exposure.

So, in summary, the following results have been observed in the present invention:
- Eeyarestatin I inhibits virus replication by targeting cellular factors (e.g. VCP/p97) and/or cellular pathways (ERAD) that are critical for a productive infection. This entails an indirect inhibitory mechanism on the virus which depends on the modulation of an intracellular host protein. The treatment with eeyarestatin I or xanthohumol leads to reduced Zika virus yields during infection in Vero cell cultures **(****Figure 3****).**
- An unexpected direct-acting antiviral activity by eeyarestatin I on Zika virus particle infectivity (virucidal activity) is shown in the present invention (**Figure 4** and **Figure 5**).
- The pre-incubation of a Zika virus sample with eeyarestatin I in the absence of cells results in a significant loss of infectivity **(****Figure 4B****).** This antiviral activity occurs outside the cell in a context where VCP/p97 is not present.
- The decay in infectivity is time- and drug concentration-dependent (**Figure 4C** and **Figure 5A**). Therefore, the drug directly affects the virus particle infectivity and/or its integrity.
- The estimated inhibitory constant is <600 nanomolar (time = 4 hours) while its 50% toxicity for Vero cells (time = 24 hours) is 24 µM (i.e. selectivity index >40) (**Figure 2A** and **Figure 5A**).
- The incubation of **Zika** virus in the presence of similar concentrations of another VCP/p97 inhibitor (xanthohumol), unrelated drug (amantadine) or vehicle (DMSO) did not affect viral infectivity (**Figure 4B** and **Figure 4C**). This constitutes further evidence supporting that eeyarestatin I exerts virucidal activity upon the viral particle, in a mechanism independent from VCP/p97 inhibition.
- Prolonged treatment with eeyarestatin I do not select for resistant Zika virus with decreased sensitivity to virucidal activity, which constitutes an advantage with respect to other direct-acting antivirals (Bhatia and Gupta, 2020).
- Eeyarestatin I, but not xanthohumol, also inhibits Usutu virus infectivity supporting that it exerts broad-acting virucidal activity against other members of the *Flaviviridae* family **(****Figure 4D** and **Figure 5B**).
- Virucidal activity by eeyarestatin I on Usutu virus is also time- and drug concentration-dependent, with an estimated IC₅₀ of 150 nanomolar (time = 4 hours), and a selectivity index of >150.

## Claims

1. Eeyarestatin I for use in the treatment of viral infections caused by a virus member of the *Flaviviridae* family.

2. Eeyarestatin I for use, according to claim 1, in the treatment of viral infections caused by a virus member of the *Flavivirus* genus.

3. Eeyarestatin I for use, according to any of the previous claims, in the treatment of viral infections caused by mosquito-borne viruses.

4. Eeyarestatin I for use, according to any of the previous claims, in the treatment of viral infections caused by Zika virus or Usutu virus.

5. Eeyarestatin I for use, according to any of the previous claims, wherein the antiviral activity is exerted by directly interacting with the viral particle during the extracellular viral phase.

6. Antiviral composition comprising eeyarestatin I and, optionally, pharmaceutically acceptable carriers or excipients, for use in the treatment of viral infections caused by a virus member of the *Flaviviridae* family.

7. Antiviral composition for use, according to claim 6, in the treatment of viral infections caused by a virus member of the *Flavivirus* genus.

8. Antiviral composition for use, according to any of the claims 6 or 7, in the treatment of viral infections caused by mosquito-borne viruses.

9. Antiviral composition for use, according to any of the claims 6 to 8, in the treatment of viral infections caused by Zika virus or Usutu virus.
